# EUROPEAN PATENT SPECIFICATION

(11) **EP 1 181 030 B1**
(45) Date of publication and mention of the grant of the patent: **10.09.2003**
(21) Application number: 00940292.6
(22) Date of filing: 31.05.2000
(51) Int. Cl.: A61K 35/78

(54) **TANACETUM PARTHENIUM EXTRACT**
TANACETUM PARTHENIUM EXTRAKT
EXTRAIT DE TANACETUM PARTHENIUM

(30) Priority: 03.06.1999 IT MI991244
(43) Date of publication of application: 27.02.2002
(73) Proprietor: INDENA S.p.A., 20139 Milano (IT)
(72) Inventor: BOMBARDELLI, Ezio, I-20141 Milano (IT); MORAZZONI, Paolo, I-20139 Milano (IT)
(74) Representative: Minoja, Fabrizio, Dr.
(86) International application number: PCT/EP00/04976
(87) International publication number: WO 00/074695

(56) References cited:
- WO-A-00/74699
- DATABASE BIOSIS [Online] BIOSCIENCES INFORMATION SERVICE, PHILADELPHIA, PA, US; 1993 BARASBY R W J ET AL: "Feverfew and vascular smooth muscle: Extracts from fresh and dried plants show opposing pharmacological profiles, dependent upon sesquiterpene lactone content." Database accession no. PREV199395123206 XP002147441 & PLANTA MEDICA, vol. 59, no. 1, 1993, pages 20-25, ISSN: 0032-0943

## Description

The present invention relates to a Tanacetum parthenium extract substantially free from α-unsaturated γ-lactons and to pharmaceutical and cosmetic compositions containing the Tanacetum parthenium extract substantially free from parthenolide.

### TECHNOLOGICAL BACKGROUND

The extracts of Tanacetum parthenium, a plant belonging to the family of Asteracee/Composite, also known under the names Altamisa, Crisanthemum, Leucanthemum, Pyrethrum parthenium or under the common name "feverfew", have traditionally been used in the treatment of migraine, vertigo, arthritis," menstrual disorders, fever, toothache, stomach ache and insect bites. The extracts of Tanacetum parthenium, in addition to volatile oils of various nature (mono- and sesquiterpene components), flavonoids, tannins, pyrethrin, also contain terpenoids of the family of sesquiterpene laccones known under the names germacranolides, guaianolides and eudesmanolides. Said compounds are characterized by an α-unsaturated γ-lactone structure and comprise in particular the compounds known under the names parthenolide, 3-β-hydroxy-parthenoide, costunolide, 3-β-hydroxy-costunolide, artemorin, 8-α-hydroxy-estafiatin and chrysanthemonin.

The presence of said sesquiterpene laccones is considered the basic condition for the pharmacological activity of the extracts (J. Pharm. Pharmacol. 1992, 44:391-395).

The attention has been focused, in particular, on parthenolide, which is thought to be the fundamental active ingredient of the extract, but also the main responsible for allergic reactions which can sometimes occur following treatment with the extracts of Tanacetum parthenium (Arch. Dermatol. Forsch. 1975, 251 (3):235-44; ibidem 1976, 255 (2):111-21; Contact Dermatitis, 1988, 38 (4):207-8; Am. J. Contact Dermatol. 1998-9 (1):49-50; Br. J. Dermatol. 1995, 132 (4): 543-7).

Extracts of Tanacetum parthenium containing parthenolide are disclosed in WO 94 06800; EP 0 553 658; WO 92 11857; GB 2,166,952; EP 98 041; WO 98 39018.

### DISCLOSURE OF THE INVENTION

It has now been found that Tanacetum parthenium extracts substantially free from α-unsaturated γ-lactons, particularly from parthenolide, have interesting pharmacological properties together with a remarkably reduced risk of allergic reactions.

The extracts of the invention can therefore be conveniently used as active ingredients for the preparation of pharmaceutical or oral cosmetic compositions.

"Substantially free from α-unsaturated γ-lactons" and "substantially free from parthenolide" herein mean an extract having a weight content of parthenolide below 0.2%, preferably below 0.1%, more preferably below 0.09% and most preferably below 0.07%.

The extract of the invention is obtainable through a process which comprises:
a) extracting the plant (aerial parts) with acetone, alcohols or mixtures of these solvents with water;
b) extracting the material from step a) with hydrocarbons;
c) extracting the non-hydrocarbon phase with an apolar solvent;
d) treating the extract with the apolar solvent, previously evaporated and redissolved in water-alcoholic solution, with a strong basic resin;
e) eluting the resin with alcohols and removing the eluted solution;
f) treating the resin with alcoholic or water-alcoholic solutions of acids, concentrating the solution and extracting the resulting residue with an apolar solvent;
g) evaporating the apolar solvent from step f) to give a residue which is added to the residue from the evaporation of the hydrocarbon extract from step b) and to the acetonic or alcoholic phase obtained after the extraction with the apolar solvent of step c) ;
h) evaporating and drying.

Parthenolide and the other related sesquiterpene lactones can be removed in that the strong basic resin surprisingly does not cause the opening of the lactone ring, which is recognizedly sensitive to basic hydrolysis: the flavone components having acid hydroxy groups are therefore retained by the resin, contrary to parthenolide and related compounds which can therefore be removed.

The preferred solvents for the various extraction steps are reported in the following:
step a): acetone, methanol, ethanol or mixtures thereof with water;
step b): hexane, n-pentane, petroleum ether, ligroin;
step c): methylene chloride, chloroform, ethyl acetate, preferably methylene chloride;
step f): ethyl acetate.
"Alcoholic or water-alcoholic solvents" preferably means methanol or methanol with water percentages ranging from 10 to 80% by volume.

Basic resins which can be used according to the invention are commercially available, for example, under the registered names Relite 2A®, Relite 3A2®, Dowex 2®.

Alternatively, the extract of the invention can be obtained starting from the conventional extracts commercially available, by treatment with strong basic resins, optionally previously extracting with hydrocarbon solvents the lipophilic components which could adversely interfere with the resin.

The content of α-unsaturated γ-lactons or of parthenolide in the final extract is determined by HPLC.

In the annexed Figure, the typical HPLC profile of the extract obtainable by the process described above is shown.

The extract of the invention has favourable pharmacological properties together with a remarkably reduced risk of inducing allergic reactions. The preservation of the pharmacological properties characteristic of the conventional extracts of Tanacetum parthenium, despite the almost complete removal of the sesquiterpene lactones, is even more surprising in view of the up-to-now documented reports as regards the relationship between bioactivity and composition of the known extracts (J. Pharm. Pharmacol. 1992, 44:391-395.).

More particularly, the extracts having a content of parthenolide below 0.2% of the invention have antiplatelet, antiinflammatory, analgesic, anti-migraine activities.

The invention therefore also relates to pharmaceutical compositions containing as active ingredient an effective amount of the Tanacetum parthenium extract of the invention in admixture with suitable carriers.

More particularly, the compositions of the invention will contain 10 to 500 mg of extract and are to be administered 3-4 times a day, depending on the physician's prescription. The preferred administration route is the oral one, but other routes, such as the topical and parenteral, could be envisaged.

The compositions of the invention are particularly useful for the therapy and prophylaxis of migraine attacks.

The extracts of the invention can also be used as active ingredients for oral cosmetic formulations.

The invention is described in greater detail in the following examples.

### EXAMPLE 1 - PREPARATION OF THE EXTRACT

Two kilograms of Tanacetum parthenium are extracted at 50-60°C with 20 L of 70% aqueous methanol. The extract is concentrated under vacuum to about 1 L and diluted with an equal volume of methanol. The resulting solution is extracted with 3 x 2 liters of n-hexane. The hexane extract is evaporated to dryness under vacuum to yield about 30 g of residue (extract H). The water-methanolic solution is then extracted with 2 x 0.5 L of methylene chloride. The organic phase is evaporated to dryness under vacuum to yield about 70 g of residues (extract DCM). The water-methanolic phase (extract HM) is set aside. The DCM extract is dissolved in 0.6 L of 90% methanol and treated under stirring with 0.6 L of strong basic resin (Relite 2A®) for three-four hours. The suspension is filtered under vacuum and the resin is washed with about 2 L of 90% methanol. The methanolic solution, containing parthenolide and its congeners, is removed. The basic resin is then treated under stirring for one hour with 0.6 L of methanol containing 65 ml of concentrated hydrochloric acid. The resin is filtered under vacuum and washed with a further 2.5 L of methanol. The filtrate and the washings are combined, concentrated under vacuum to about 200 mL and extracted with 3 x 200 ml of ethyl acetate. The resulting extract (E.A.), containing the flavone components tanetin and congeners, is evaporated to dryness under vacuum, to obtain about 4 g of a residue. The residues of the extracts H and E.A. are combined with the extract HM. The resulting solution is evaporated to dryness under vacuum and the solid residue is dried under vacuum at 50°C to constant weight. About 490 g of extract of departhenolidized T. parthenium are obtained which shows, by HPLC analysis, (column Zorbax SB C18; eluent H₂O + 0.01% TFA; B:MeCN + 0.01% TFA; gradient A:B:90%-10%:10%-90%; flow 1 ml/min), a parthenolide content below 0.07%.

### EXAMPLE 2 - PHARMACOLOGICAL ACTIVITY

The experimental model of nitroglycerin-induced neuronal stimulation in the rat, described in Brain Research 1995,682:167-181; *ibidem* 1995 695,37-44 and in Neuropharmacol. 1997, 36(10);1417-1424, has been used.

Nitroglycerin (a vasodilator commonly used to treat some cardiovascular disorders) can be used as a diagnostic tool in the study of migraine headache, since its administration causes a spontaneous-like attack in migraieurs. The nitroglycerin-induced attack is not immediate, but delayed by some hours and typically associated with the accompanying symptoms like nausea and photophobia. The vasodilating activity of organic nitrates, including nitroglycerin, arises from the metabolism to nitric oxide which is also reported to play a role as a neurotransmitter in the SNC. Neuronal stimulation rapidly activates the transcription of several proto-oncogenase, which, because of their rapid and transient induction, have been named immediate-early genes. Among them, c-fos is the most extensively studied and the protein encoded, Fos, is translocated to the nucleus where it persists for a few hours and can be detected immunoistochemically.

### Experimental procedures

Twenty-five male Sprague-Dawley rats divided in 5 animal group each have been used. Two groups of animals (control and nitroglycerin) were injected with vehicle alone (0.5% carboxymethylcellulose, 10 ml/kg) while the 3 other groups were treated orally for 3 days with a Tanacetum parthenium extract substantially free from parthenolide (TPPDE) at the doses of 100 and 200 mg/kg and with Tanaceutum parthenium regular extract (TPRE) at the same dosages, respectively.

One hour after the last administration, all the animals, except the control group, were treated subcutaneously with 10 ml/kg of nitroglycerin.

Four hours after administration of nitroglycerin, the rats were anaesthetized with sodium pentobarbital and perfused with fixative through the ascending aorta. The brains were quickly removed and postfixed in the same fixative overnight. Sections through the entire brain were cut at 50 µm on a freezing sliding microtome. Sections were serially collected in six wells containing cold phosphate buffered saline (PBS) and processed for immunoistochemistry as free-floating sections.

The results are reported in the following Table.

TABLE: Number of immunoreactive neurons (mean ± s.d.) in the paraventricular nucleus of the hypothalamus (PVH), locus coeruleus (LC), and parabrachial nucleus (PBN) 4 hours after nitroglycerin administration.

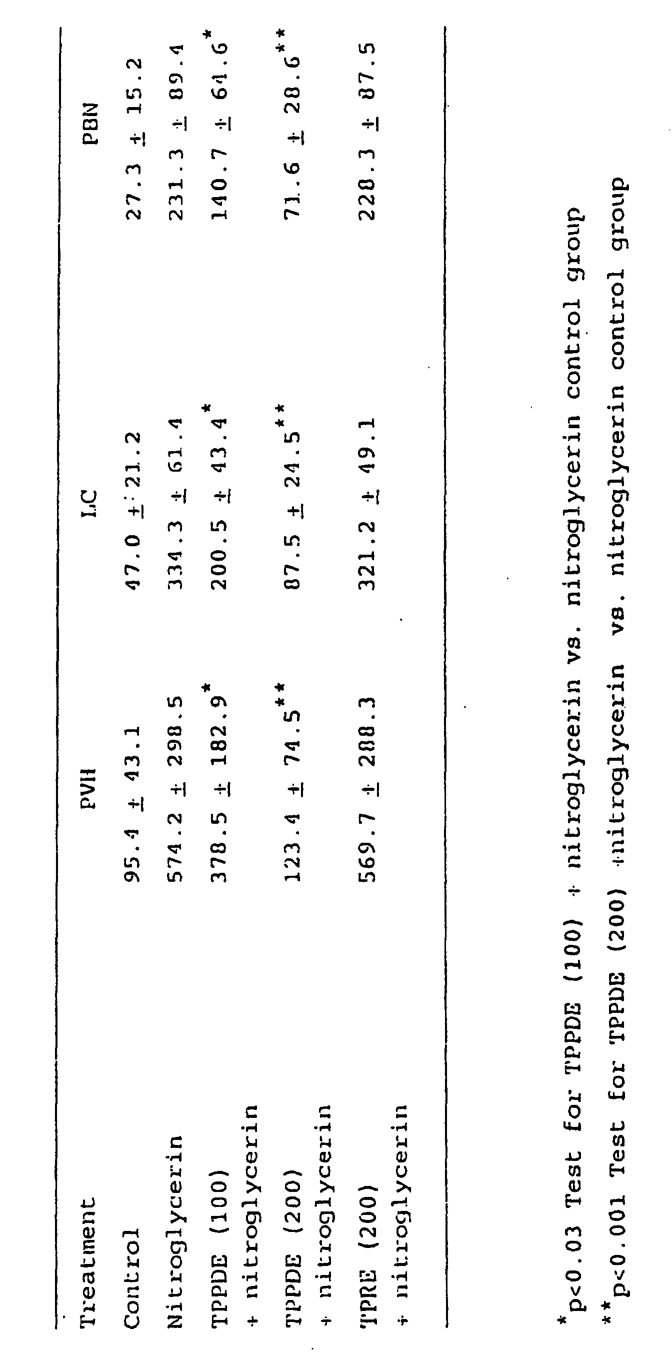

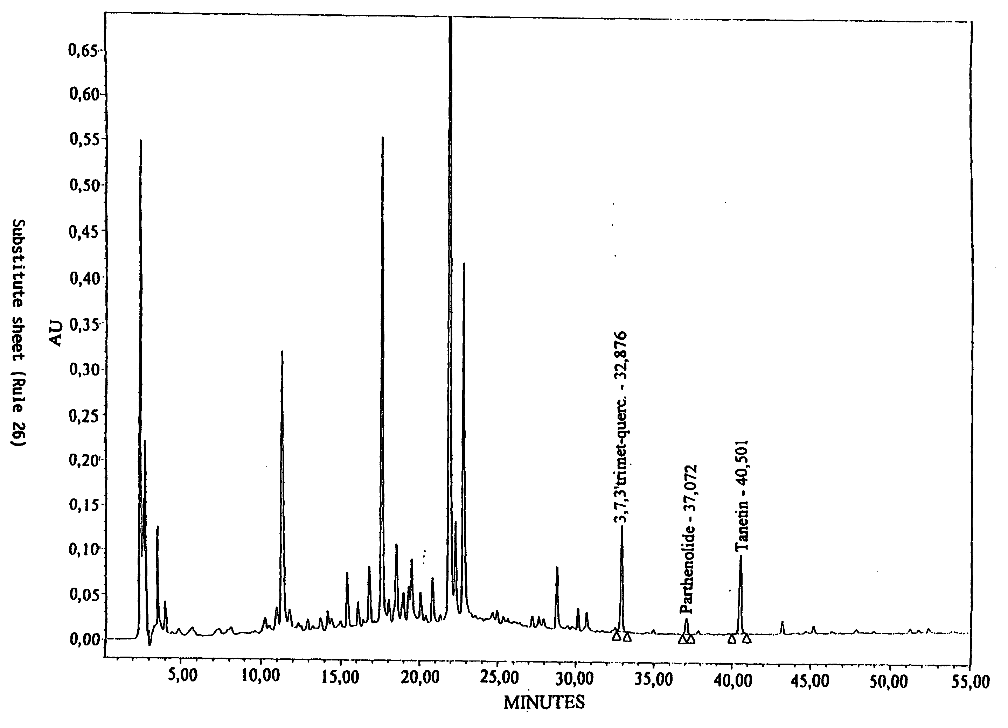

## Claims

1. Extracts of Tanacetum parthenium having a content of α-unsaturated γ-lactons below 0.2% obtainable by a process which comprises:
a) extracting the plant (aerial parts) with acetone, alcohols or mixtures of these solvents with water;
b) extracting the material from step a) with hydrocarbons;
c) extracting the non-hydrocarbon phase with an apolar solvent;
d) treating the extract with the apolar solvent, previously evaporated and redissolved in water-alcoholic solution, with a strong basic resin;
e) eluting the resin with alcohols and removing the eluted solution;
f) treating the resin with alcoholic or water-alcoholic solutions of acids, concentrating the solution and extracting the resulting residue with an apolar solvent;
g) evaporating the apolar solvent from step f) to give a residue which is added to the residue from the evaporation of the hydrocarbon extract from step b) and to the acetonic or alcoholic phase obtained after the extraction with the apolar solvent of step c);
h) evaporating and drying.

2. An extract according to claim 1 having a content of parthenolide below 0.2%.

3. An extract according to claim 1 or 2 having a content of α-unsaturated γ-lactons below 0.1%.

4. Pharmaceutical compositions containing the extract of claims 1-3 in admixture with suitable carriers.

5. Oral cosmetic compositions containing as active ingredient the extracts of claims 1-3 in admixture with suitable carriers.

## Patentansprüche

1. Extrakte von Tanacetum parthenium mit einem Gehalt an α-ungesättigten γ-Lactonen unter 0,2%, erhältlich durch ein Verfahren, das umfasst:
a) Extrahieren der Pflanze (oberirdische Teile) mit Aceton, Alkoholen oder Gemischen von diesen Lösungsmitteln mit Wasser;
b) Extrahieren des Materials von Schritt a) mit Kohlenwasserstoffen;
c) Extrahieren der Nicht-Kohlenwasserstoff-Phase mit einem unpolaren Lösungsmittel;
d) Behandeln des Extrakts mit dem unpolaren Lösungsmittel, vorher eingedampft und erneut in Wasser-alkoholischer Lösung gelöst, mit einem stark basischen Harz;
e) Eluieren des Harzes mit Alkoholen und Entfernen der eluierten Lösung;
f) Behandeln des Harzes mit alkoholischen oder Wasseralkoholischen Lösungen von Säuren, Einengen der Lösung und Extrahieren des erhaltenen Rückstands mit einem unpolaren Lösungsmittel;
g) Eindampfen des unpolaren Lösungsmittels von Schritt f) zur Gewinnung eines Rückstandes, der zu dem Rückstand aus der Verdampfung des Kohlenwasserstoffextrakts von Schritt b) und zu der acetonischen oder alkoholischen Phase, die nach der Extraktion mit dem unpolaren Lösungsmittel von Schritt c) erhalten wurde, gegeben wird;
h) Eindampfen und Trocknen.

2. Extrakt nach Anspruch 1 mit einem Gehalt an Parthenolid unter 0,2%.

3. Extrakt nach Anspruch 1 oder 2 mit einem Gehalt an α-ungesättigten γ-Lactonen unter 0,1%.

4. Pharmazeutische Zusammensetzungen, die den Extrakt von Ansprüchen 1-3 in Anmischung mit geeigneten Trägern enthalten.

5. Orale kosmetische Zusammensetzungen, die als Wirkstoff die Extrakte von Ansprüchen 1-3 in Anmischung mit geeigneten Trägern enthalten.

## Revendications

1. Extraits de *Tanacetum parthenium* ayant une teneur en γ-lactones α-insaturées inférieure à 0,2 % pouvant être obtenus par un procédé qui comprend les étapes suivantes :
a) extraction de la plante (parties aériennes) avec l'acétone, des alcools ou des mélanges de ces solvants avec l'eau ;
b) extraction de la matière venant de l'étape a) avec des hydrocarbures ;
c) extraction de la phase non hydrocarbonée avec un solvant apolaire ;
d) traitement de l'extrait obtenu avec le solvant apolaire, préalablement évaporé et redissous dans une solution hydro-alcoolique, avec une résine basique forte;
e) élution de la résine avec des alcools et élimination de la solution éluée ;
f) traitement de la résine avec des solutions alcooliques ou hydro-alcooliques d'acides, concentration de la solution et extraction du résidu résultant avec un solvant apolaire ;
g) évaporation du solvant apolaire venant de l'étape f) pour obtenir un résidu qui est ajouté au résidu venant de l'évaporation de l'extrait hydrocarboné venant de l'étape b) et à la phase acétonique ou alcoolique obtenue après l'extraction avec le solvant apolaire de l'étape c) ;
h) évaporation et séchage.

2. Un extrait selon la revendication 1, ayant une teneur en parthénolide inférieure à 0,2 %.

3. Un extrait selon la revendication 1 ou 2, ayant une teneur en γ-lactone α-insaturées inférieure à 0,1 %.

4. Compositions pharmaceutiques contenant l'extrait des revendications 1 à 3 en mélange avec des supports appropriés.

5. Compositions cosmétiques orales contenant, comme ingrédient actif, les extraits des revendications 1 à 3 en mélange avec des supports appropriés.
